Europäisches Patentamt

European Patent Office

Office européen des brevets

(19)

(11) EP 0 975 446 B1

(12) **EUROPEAN PATENT SPECIFICATION**

(45) Date of publication and mention
of the grant of the patent:
**15.10.2003 Bulletin 2003/42**

(51) Int Cl.⁷: **A61B 5/00**, A61B 5/024

(21) Application number: **97906321.1**

(22) Date of filing: **21.03.1997**

(86) International application number:
**PCT/IB97/00290**

(87) International publication number:
**WO 98/042250 (01.10.1998 Gazette 1998/39)**

(54) **METHOD AND APPARATUS FOR MEASURING PULSE RATE AND SATURATION**

VERFAHREN UND GERÄT ZUR MESSUNG VON PULS UND SÄTTIGUNG

PROCEDE ET APPAREIL DE MESURE DE FREQUENCE DE POULS ET DE SATURATION

(84) Designated Contracting States:
**AT BE CH DE DK ES FI FR GB GR IE IT LI LU MC NL PT SE**

(43) Date of publication of application:
**02.02.2000 Bulletin 2000/05**

(73) Proprietor: **Nellcor Puritan Bennett Incorporated Pleasanton, CA 94588 (US)**

(72) Inventors:
• **Baker, Clark R., Jr.**
**Castro Valley, CA 94546 (US)**
• **Yorkey, Thomas J.**
**San Ramon, CA 94583 (US)**

(74) Representative: **Belcher, Simon James**
**Urquhart-Dykes & Lord**
**Tower House**
**Merrion Way**
**Leeds LS2 8PA (GB)**

(56) References cited:
**EP-A- 0 335 357**     **EP-A- 0 555 553**
**WO-A-95/10038**     **WO-A-97/00041**
**DE-A- 3 146 063**

• **NEHORAI A ET AL: "ADAPTIVE COMB FILTERING FOR HARMONIC SIGNAL ENHANCEMENT" IEEE TRANSACTIONS ON ACOUSTICS, SPEECH AND SIGNAL PROCESSING, vol. ASSP-34, no. 5, October 1986, pages 1124-1138, XP000676140 cited in the application**
• **HENDRY S D: "COMPUTATION OF HARMONIC COMB FILTER WEIGHTS" IEEE TRANSACTIONS ON SIGNAL PROCESSING, vol. 41, no. 4, April 1993, pages 1677-1680, XP000676141 cited in the application**

**Description**

This invention relates to a method and apparatus for measuring physiological parameters, in particular for reducing noise effects, including effects arising from motion, in a system for measuring the pulse rate of a patient. It relates in particular to a method and apparatus for measuring pulse rate and saturation. The invention involves the application of filtering techniques.

It is known to determine pulse rate from the optical plethysmograph waveform by counting zero crossings. If there is no motion and no other noise, the optical plethysmograph signal is very clean and a pulse rate can be determined by accurately counting zero crossings. During motion, a zero-crossing approach will count transients from the motion and display an artificially high rate.

It is also known to determine pulse rate using a template of the plethysmograph signal corresponding to a single pulse, and then counting the number of matches with this template. However, motion effects can lead to the appearance of the signal differing significantly from that of the template; as a result, no pulse counting occurs.

The present invention provides a technique for reducing noise effects in a system for measuring pulse rate, by use of a comb filter to isolate signal energy which corresponds to fundamental and related frequencies.

Accordingly, in one aspect, the invention provides a method of measuring a pulse rate of a patient using data corresponding to at least one wavelength of electromagnetic energy transmitted through tissue of the patient, the method comprising the steps of:

   (a) defining a comb filter to isolate signal energy in the data corresponding to a fundamental frequency of the pulse rate and related higher frequency components thereof;

   (b) determining a particular frequency which optimizes energy at the output of the comb filter; and

   (c) generating a filtered pulse rate corresponding to the particular frequency.

In another aspect, the invention provides apparatus for measuring a pulse rate of a patient using data corresponding to at least one wavelength of electromagnetic energy transmitted through tissue of the patient, comprising:

   (a) a comb filter for isolating signal energy in the data corresponding to a fundamental frequency of the pulse rate and related higher frequency components thereof;

   (b) means for determining a particular frequency which optimizes energy at an output of the comb filter; and

   (c) means for generating a filtered pulse rate corresponding to the particular frequency.

The technique of the present invention estimates the frequencies and amplitudes of sinusoids in noise. A procedure to find the frequencies and then the amplitudes and phases of a given number of arbitrary sinusoids in noise is disclosed in *A Minimal Parameter Adaptive Notch Filter with Constrained Poles and Zeros* (Nehorai A), volume 33 of the IEEE Transactions on Acoustics, Speech, and Signal Processing (1985). This approach has been extended specifically to look for the fundamental frequency and then the amplitudes and phases of a harmonic signal in noise, as disclosed in *Adaptive Comb Filtering for Harmonic Signal Enhancement* (Nehorai A and Porat B), volume 34 of the IEEE Transactions on Acoustics, Speech, and Signal Processing (1986). A numerically more efficient procedure for finding the fundamental frequency based on this approach is the subject of *Computation of Harmonic Comb Filter Weights* (Hendry S D), volume 41 of the IEEE Transactions on Acoustics, Speech, and Signal Processing (1993).

WO-A-95/10038 discloses a method and an apparatus for measuring a pulse rate of a patient using data corresponding to at least one wavelength of electromagnetic radiation transmitted through tissue of the patient, and comprising (defining) a comb filter and generating a filtered pulse rate.

The technique employed by the present invention is referred to herein as Adaptive Comb Filtering (ACF). The pulse rate is calculated from the optical absorbance signal.

When viewed as a spectrogram (frequency versus time), the energy in a typical human plethysmograph signal is distributed in a few stable, clearly defined bands corresponding to the harmonics of the pulse rate. Adaptive comb filtering first finds the fundamental frequency by defining a comb filter to notch out the energy of the signal, leaving only noise. The number of notches or signal harmonics in the signal is a matter of choice. For normal heart rates, four harmonics can be preferred, but other numbers may be preferred depending on the application, processor, computation power, and the low pass cutoff frequency of a bandpass filter. The ACF finds the harmonic frequency that defines the comb filter to cause the output noise outside the fundamental and chosen harmonics to have the smallest energy. The ACF searches for the fundamental by estimating derivatives of the squared noise with respect to the fundamental to perform a Newton-Raphson search which is the classic approach to nonlinear minimization as discussed in the Nehorai and Porat (1986) and Hendry (1993) documents referred to above.

To formalize the minimization description, let y be the measured signal, *x* the harmonic signal, and *n* the noise

$$y(t) = x(t) + n(t)$$

$$= \sum_{k=1}^{N} C_k \sin(k\omega t + \phi_k) + n(t)$$

In the z-transform domain, the comb filter is

$$H(z^{-1}) = \frac{\displaystyle\prod_{k=1}^{N} (1 + \alpha_k z^{-1} + z^{-2})}{\displaystyle\prod_{k=1}^{N} (1 + \rho\alpha_k z^{-1} + \rho^2 z^{-2})}$$

where

$$\alpha_k = -2 \cos k\omega_0$$

The parameter $\omega_0$ is the fundamental frequency normalized by the sample rate. Each quadratic in the numerator of *H* introduces a zero on the unit circle at $\pm k\omega_0$. With $\rho < 1$, the denominator introduces a pole inside the unit circle at $\pm k\omega_0$. The bandwidth of the notches is $\pi(1-\rho)$.

When a reliable estimate of the fundamental frequency has been determined, it is preferred to at least occasionally calculate the harmonic amplitudes. A description of a technique for calculating the harmonic amplitudes follows.

It is useful to know the amplitudes of the harmonics. The power of each of the first n = 4 harmonics is estimated by taking an RMS of the output of a comb filter having only one "tooth" tuned to the frequency of that harmonic. This allows the power of the harmonics to be estimated with a minimal number of computations. This process can be performed independently of the adaptive comb filter provided the heart rate estimate is made available to this process. For each harmonic k, the steps in the harmonic estimation are:

1. Calculate *a* for $k\omega_0$

$$a_1 = -2\cos k\omega_0, \ a_0 = a_2 = 1$$

2. Calculate the output of the "single toothed" comb filter at $k\omega_0$

$$\varepsilon(t) = y(t) + \sum_{i=1}^{2} y(t-i) a_i - \sum_{i=1}^{2} \overline{\varepsilon}(t-i) \rho^i a_i$$

3. Estimate the bandpass gain of the "single toothed" comb filter at $k\omega_0$ as the gain at DC

$$K(t) = \frac{\sum_{i=0}^{2} a_i}{\sum_{i=0}^{2} \rho^i a_i}$$

4. Estimate the harmonic signal at $k\omega_0$ as the difference between the measured signal and the output of the "single toothed" comb filter.

$$x(t) = y(t) - \varepsilon(t)/K(t)$$

5. Estimate the power (pwr) in the harmonic at $k\omega_0$ using a cascaded IIR filter

$$pwr(t) = \lambda pwr(t-1) + (1-\lambda)x(t)^2$$

$$pwr'(t) = \lambda pwr'(t-1) + (1-\lambda)pwr(t)^2$$

where $\lambda$ represents the degree of acceptable variability

There are times when the ACF by itself is not sufficient to track a patient's heart rate. This is the case with arrhythmias, where the energy is not concentrated in a few trackable harmonics. Also, a patient's heart rate can change more rapidly than the ACF is capable of tracking, or change dramatically during a period of motion in which the ACF failed to correctly track the change. An alternative pulse rate calculator is therefore included in the present invention that does not require a harmonic signal, and is not based on an adaptive algorithm. This alternative rate calculator is not as robust during motion as the rate calculator described above, but is intended to be used when the ACF cannot track the rate.

A feature that is common to all human pulses is that they go up and down and have some minimum period corresponding to about 250 beats per minute, i.e., 240 msec. Given this model of a human pulse, there is a crest during each pulse such that the current value of the pulse waveform is the maximum value that has been seen for the past 240 msec followed by a trough during that same pulse where the current value is the minimum value that has been seen for the past 240 msec. The pattern matcher provided by the present invention identifies sequences of crests and troughs that preferably do not trigger any motion detection criterion (discussed below), and computes the rate from the average period of all such patterns which have been identified in the past 10 seconds. In one embodiment, the pattern matcher uses a minimum period of 210 msec instead of 240 msec to make allowances for limited motion artifact and an oximeter sampling rate of about 57 Hz. The pattern matching rate is updated each time such a pattern is identified. In some preferred embodiments, motion detection criterion based on the shape of the pulse have been adapted to reject pulses which are potentially contaminated by motion artifact.

Preferably, the step referred to above of determining a pattern matching pulse rate includes the steps of:

(a) identifying a sequence of waveform characteristics indicative of a waveform period;
(b) averaging a number of successive waveform periods to determine an average waveform period; and
(c) determining the alternate pulse rate from the average waveform period.

The method can include a step of comparing the data to a predetermined waveform template prior to the identification step.

Preferably, the motion detection criteria require the calculation of the maxima, minima, and average for the current and previous detected patterns. Motion is detected when any of the following criteria are met:

a) the maximum of the current pattern is significantly further away from the average of the current pattern than the minimum is;
b) criterion a) failed on the last detected pattern;
c) the maximum of the current pattern is significantly further away from the average of the current pattern than the average of the current pattern is from the average of the current and previous minima; or

d) the difference between the average of the current and previous patterns is greater than half the difference between the current maximum and minimum (big DC shift).

A motion flag is set whenever any of the motion detection criteria are met and is cleared whenever none are met for two consecutive patterns.

In a preferred embodiment, two pattern matching rate calculators are run in parallel. One receives the bandpassed normalized waveform as input. The second receives a filtered form of the first derivative of the plethysmograph. The use of two inputs with different characteristics minimizes the time that motion is incorrectly reported. Each pattern matcher stores the proportion of patterns for which its motion metrics indicate motion. The pattern matcher that reports the least motion over the last ten seconds is used for the pattern matching rate. This dual pattern matcher reports that motion is present only when the motion metrics for each of its two pattern matchers indicates motion.

As discussed above, the adaptive comb filter (ACF) employed by the present invention tracks a signal having N harmonics. It sometimes happens that motion artifact causes the ACF to track the wrong rate, and when the motion stops, the "teeth" on the comb may be on a harmonic rather than the fundamental. For example, the fundamental of the ACF could be tracking the second harmonic of the plethysmograph, or the second harmonic for the ACF could be tracking the fundamental of the plethysmograph. These situations would cause the ACF to report twice or half the correct pulse rate, respectively. This could happen if the ACF was initialized to the wrong rate and settled on a harmonic or subharmonic, or if the rate changed more suddenly than the ACF could track. In general, the ACF is quite stable, and several minutes of prolonged, vigorous motion may be required before it locks onto a harmonic or subharmonic of the pulse rate. However, because of the potential for false reporting, according to a preferred embodiment a number of rules are used to calculate a more accurate rate.

A simple model of most plethysmographs may be compared to a sawtooth-like pattern for which the amplitude of the harmonics of the plethysmograph fall off by a factor of two for each harmonic. Thus, for most plethysmographs, the falloff is fairly rapid. However, some patients have nearly half the energy of their pulses contained in the second harmonic. Other physiological plethysmograph patterns may contain significant amounts of energy at multiples of one-half the pulse rate, while still others may have strong respiratory components at one-half the pulse rate, although the signal at the pulse rate should still be dominant. Arrhythmias may have no repeating pattern, thus violating the model assumed by the ACF. When the ACF locks onto a subharmonic, or some superharmonic, the "tooth" on the comb that passes the greatest amount of energy will not correspond to the fundamental frequency estimated by the ACF.

For most patients, where there is little or no motion, all the energy in the plethysmograph is at the fundamental or a harmonic of the pulse rate. Although pulse rates will vary cyclically in response to various mechanisms, plateaus in the pulse rate will be long enough, and frequent enough, that the autocorrelation has a very high value at the fundamental of the pulse rate at these times. Where there is significant energy at a subharmonic of the pulse rate, the autocorrelation at the subharmonic may be higher than at the pulse rate, but the autocorrelation function will still have a strong local maxima at the pulse rate.

For an arrhythmia with a non-repeating pattern, the autocorrelation may not have any strong local maxima. However, if a patient is not moving, the plethysmograph will be modulated only by the patient's pulse, and will thus be completely correlated in the IR and red channels. Motion artifact, however, causes the IR and red channels to become less correlated. This crosscorrelation can thus be used as a rough indicator of the degree of motion and therefore the reliability of the rate reported by the pattern matching.

In view of the foregoing, a concise set of rules is desirable that reliably enables the ACF to resume tracking the correct pulse rate when motion has stopped, and which does not increase the chance that ACF will track the wrong pulse rate. An "uncorrelation" metric is calculated which is defined as:

$$\sqrt{1\text{-}crosscorrelation(IR, red)^2},$$

where the crosscorrelation is currently calculated over about 10 seconds of normalized data.

The magnitude of each of the harmonics is estimated each second, and the magnitude information is used to calculate a measure of the validity of the pulse. In addition to estimating the pulse rate, the ACF also calculates filtered signal and noise waveforms, X and $\in$ using the comb filter, which enables the calculation of a signal to noise (S/N) ratio. The S/N ratio is expected to be high for non-arrhythmic patients who are not moving, and low during motion or arrhythmias. It is also low if the fundamental of the heart rate does not match any of the harmonics being tracked by the ACF. In various embodiments, the S/N may be calculated by differentiating, filtering in derivative space, and then integrating the plethysmographs, with the best S/N ratio being used.

A preferred valid plethysmograph will have the energy in consecutive harmonics falling off exponentially. If a majority of the energy in the plethysmograph is calculated to be in one of the harmonics instead of the fundamental, the standard deviation for the exponential decay will probably be large enough to drive the validity measure negative.

The following mechanisms can be used to assure that the ACF is tracking the right rate. A Fast Fourier Transform (FFT) (power spectrum) can be performed on the IR plethysmograph and each peak in the spectrum may be evaluated prospectively to find the rate which would give the highest confidence measure for the ACF (the formulas used to combine various metrics into a confidence measure for the ACF rate are described below). This is possible because the magnitude of each of the harmonics that would be tracked by the ACF at a given rate can be estimated by adding up the energy in several adjacent frequencies of the FFT.

The ACF rate is reset to this FFT rate if the FFT rate is considered more reliable than the ACF rate. Ideally, the ACF rate should not be reported until it has been initialized by this mechanism.

The determining step can minimise or maximise the energy at the output of the comb filter, according to the selected operation of the ACF .

Preferably, the determining step of the method of the invention comprises:

(a) calculating squared noise for the data;
(b) calculating a second derivative of the squared noise with respect to the fundamental frequency; and
(c) performing a Newton-Raphson search to determine the particular frequency.

Preferably, the method includes the steps of:

(a) comparing selected samples of the pulse rate to an expected measurement characteristic;
(b) assigning a variable weight to each selected sample based on the comparing step, thereby generating a plurality of differently weighted samples of the pulse rate;
(c) averaging a first number of the weighted samples to obtain a filtered sample of the pulse rate, the first number preferably varying in response to the variable weights assigned in the assigning step; and
(d) generating a filtered pulse rate from a plurality of filtered samples.

Preferably, before the defining step of the method, it includes the steps of:

(a) taking the logarithm of a signal representative of at least one wavelength of electromagnetic energy, thereby generating a first signal;
(b) band pass filtering the first signal, thereby generating a second signal;
(c) normalizing the second signal, thereby generating a third signal; and
(d) taking the derivative of the third signal, thereby generating the data.

There will generally be a confidence level associated with the pulse rate, the confidence level being based on at least one quality metric selected from the group comprising pulse signal shape, signal-to-noise ratio, and correlation between at least two wavelengths of electromagnetic energy.

Preferably, the method includes the steps of:

(a) determining an alternate pulse rate using an alternate rate finder; and
(b) selectively setting the pulse rate to the alternate pulse rate.

The alternate pulse rate can be determined by:

(a) evaluating a power spectrum corresponding to the data to determine which of a plurality of peaks in the power spectrum corresponds to the fundamental frequency;
(b) generating an alternate pulse rate corresponding to the fundamental frequency; and
(c) calculating a confidence level for the alternate pulse rate.

The method can include the step of generating an initial pulse rate corresponding to the alternate pulse rate in the setting step.

The step of determining an alternate pulse rate can be arranged to occur periodically, the setting step only then occurring where at least one condition is met. That condition can be selected from the group comprising:

(a) a confidence level corresponding to the filtered pulse rate is less than a first value;
(b) the confidence level corresponding to the alternate pulse rate is greater than or equal to the first value;
(c) a signal-to-noise ratio corresponding to the alternate pulse rate is greater than or equal to a second value;
(d) the alternate pulse rate corresponds to a local maxima on an autocorrelation curve corresponding to the data; and

(e) the alternate pulse rate is within a third value of a previously determined alternate pulse rate, the previously determined alternate pulse rate having been determined within a first interval.

The method can be performed such that conditions (a) to (e) above are met before the pulse rate is set to the alternate pulse rate.

The technique of the present invention can be used in conjunction with a technique for calculating oxygen saturation levels in haemoglobin, providing information relating to pulse rate.

The invention can involve reduction of noise effects when measuring a physiological parameter. It can include apparatus for reducing the noise effects which comprises:

means for generating a plurality of measurements derived from at least one wavelength of electromagnetic energy transmitted through living tissue;

means for providing a signal indicative of the at least one wavelength of electromagnetic energy;

means for comparing selected measurements with at least one expected measurement characteristic;

means for assigning one of a plurality of variable weights to each selected measurement based on the comparing step thereby generating a plurality of differently weighted measurements for each wavelength, the variable weights being assigned, in part, in response to a similarity between each selected measurement and a corresponding previous measurement, the variable weights comprising a plurality of different non-zero numbers;

means for averaging a plurality of the differently weighted measurements to obtain a filtered measurement for use in estimating the physiological parameter; and

means for calibrating the system to measure the physiological parameter in response to the signal indicative of the at least one wavelength of electromagnetic energy.

The invention is useful in connection with a monitor for measuring a physiological parameter, the monitor being for use with a sensor having emitting means for emitting at least one wavelength of electromagnetic energy, sensing means for sensing the electromagnetic energy and for generating a first signal representative thereof, means for detachably coupling the sensor to the oximeter and for providing communication of signals between the sensor and the oximeter, and means for providing a second signal indicative of the at least one wavelength of electromagnetic energy, the monitor comprising:

means for generating a plurality of measurements derived from the first signal;

means for comparing selected measurements with at least one expected measurement characteristic;

means for assigning one of a plurality of variable weights to each selected measurement based on the comparing step thereby generating a plurality of differently weighted measurements, the variable weights being assigned, in part, in response to a similarity between each selected measurement and a corresponding previous measurement, the variable weights comprising a plurality of different non-zero numbers;

means for averaging a plurality of the differently weighted measurements to obtain a filtered measurement for use in estimating the physiological parameter; and

means for calibrating the monitor to measure the physiological parameter in response to the second signal.

The present invention will now be described, by way of example only, with reference to the accompanying drawings, in which:

Figure 1 is a schematic representation of apparatus for measuring a physiological parameter such as oxygen saturation of haemoglobin of a patient;

Figure 2 is a block diagram illustrating the flow of data in apparatus such as that shown in Figure 1;

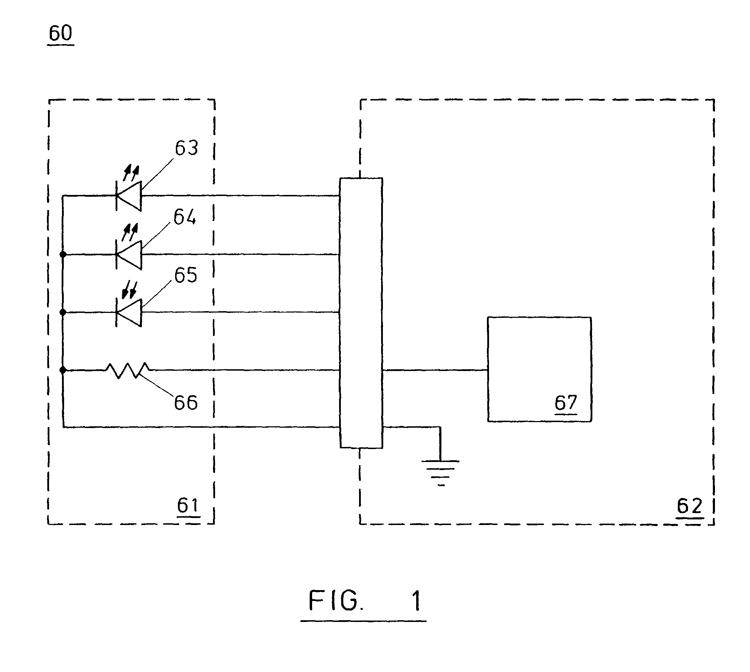

Referring to the drawings, Figure 1 shows apparatus for measuring physiological parameters such as oxygen saturation of haemoglobin of a patient. A sensor/oximeter combination 60 comprises a sensor 61 and an oximeter monitor 62. Sensor 61 includes LEDs 63 and 64 typically having wavelength emission characteristics in the infrared and red ranges of the spectrum, respectively. Photodiode sensor 65 receives the light transmitted by LEDs 63 and 64. Resistor 66 (or a similar electrical impedance reference) is chosen to correspond to a specific wavelength or combination of wavelengths as specified by a table relating impedance values to wavelengths. Decoding means 67 determines the impedance value of resistor 66, and appropriate extinction coefficients are generated which correspond to the transmission characteristics of the particular sensor 61. Thus, the oximeter may be used with a variety of sensors having LEDs which emit varying wavelengths of light without recalibration. The sensor 61 is detachably coupled to oximeter monitor 62 via connector 68. An example of such a sensor/oximeter combination is disclosed in US-4621643.

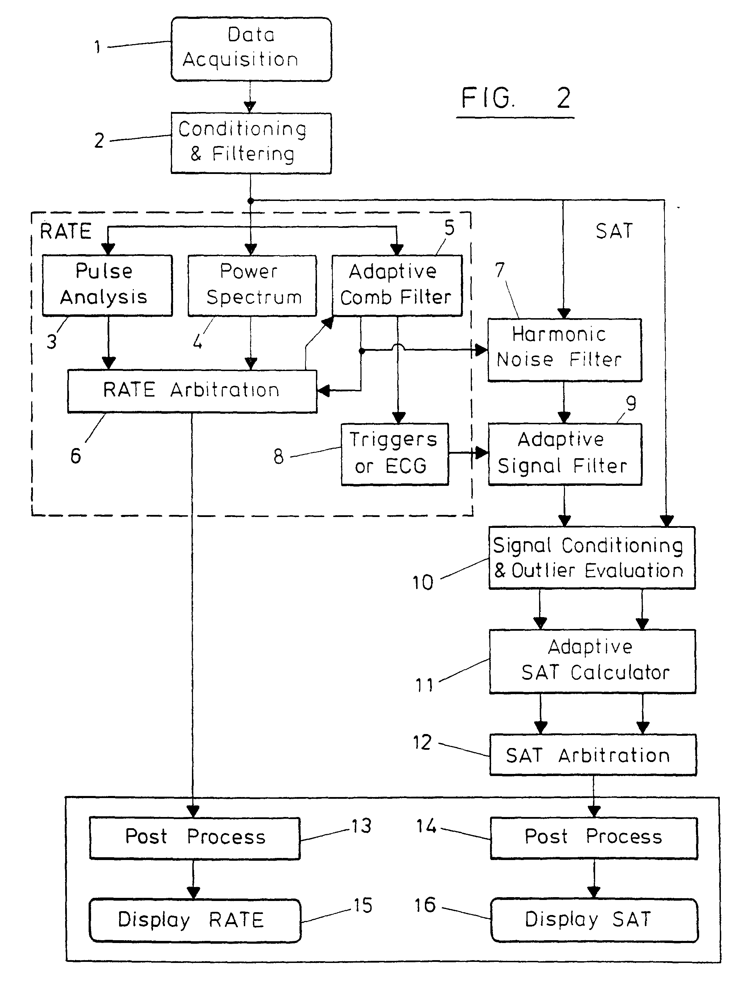

The data received from the sensor is processed according to the scheme shown in Figure 2. It can be processed using apparatus of the type disclosed in US-5348004. In initial process step 2, the natural logarithm of the data (usually from red and infra red LEDs) is taken, and the data is band pass filtered with an infinite impulse response (IIR) filter that has a high pass cutoff frequency at 0.5 Hz (that is 30 beats per minute) and a low pass rolloff from 10 to 20 Hz. The filtered data can then processed by algorithms for calculation of pulse rate as discussed in detail above.

In the illustrated arrangement, data are sent to two different algorithms for calculation of the patient's pulse rate. According to one algorithm, the derivative of the data is taken 2 as described above, and the fundamental frequency of the pulse rate is tracked using an adaptive comb filter (ACF) 5 as discussed below. The ACF 5 supplies its pulse rate directly to a harmonic filter 7. The ACF 5 can also provide a trigger for an algorithm for calculating oxygen saturation as disclosed in the related application referred to above. ACF 5 is a robust pulse rate tracker, but not a good pulse rate finder. Therefore, the frequency power spectrum of the data is calculated periodically 4 to determine whether ACF 5 is tracking the fundamental rather than a super- or subharmonic of the pulse rate.

The normalized data is also supplied to a pattern matching algorithm 3 which recognizes sequences of crests and troughs in the data and calculates an average period of the plethysmograph over a set number of samples. This algorithm is preferably used primarily to track the pulse rate for an arrhythmic pulse rate during periods where no motion is detected. A rate arbitration algorithm 6 then arbitrates between the pulse rates calculated by ACF 5 (as updated by frequency power spectrum 4) and pattern matching algorithm 3 using confidence levels associated with each, which are based on various metrics. After appropriate post processing 13, the pulse rate data is output to the display 15. The data is post processed using available metrics to determine the reliability of the pulse rate and to determine whether and how it is to be displayed.

**Claims**

1. A method of measuring a pulse rate of a patient using data corresponding to at least one wavelength of electromagnetic energy transmitted through tissue of the patient, the method comprising the steps of:

   (a) defining a comb filter to isolate signal energy in the data corresponding to a fundamental frequency of the pulse rate and related higher frequency components thereof;

   (b) determining a particular frequency which optimizes energy at an output of the comb filter; and

   (c) generating a filtered pulse rate corresponding to the particular frequency.

2. A method as claimed in claim 1, in which the determining step minimizes the energy at the output of the comb filter.

3. A method as claimed in claim 1, in which the determining step maximizes the energy at the output of the comb filter.

4. A method as claimed in claim 1, in which the determining step comprises:

   (a) calculating squared noise for the data;

   (b) calculating a second derivative of the squared noise with respect to the fundamental frequency; and

   (c) performing a Newton-Raphson search to determine the particular frequency.

5. A method as claimed in claim 1, which includes the steps of:

   (a) comparing selected samples of the pulse rate to an expected measurement characteristic;

   (b) assigning a variable weight to each selected sample based on the comparing step, thereby generating a plurality of differently weighted samples of the pulse rate;

   (c) averaging a first number of the weighted samples to obtain a filtered sample of the pulse rate; and

   (d) generating a filtered pulse rate from a plurality of filtered samples.

6. A method as claimed in claim 5, in which the first number varies in response to the variable weights assigned in the assigning step.

7. A method as claimed in claim 1, in which, preceding the defining step, the method further comprises the steps of:

    (a) taking the logarithm of a signal representative of the at least one wavelength of electromagnetic energy, thereby generating a first signal;

    (b) band pass filtering the first signal, thereby generating a second signal;

    (c) normalizing the second signal, thereby generating a third signal; and

    (d) taking the derivative of the third signal, thereby generating the data.

8. A method as claimed in claim 1, in which there is a confidence level associated with the pulse rate, the confidence level being based on at least one quality metric selected from the group comprising pulse signal shape, signal-to-noise ratio, and correlation between at least two wavelengths of electromagnetic energy.

9. A method as claimed in claim 1, which includes the steps of:

    (a) determining an alternate pulse rate using an alternate rate finder; and

    (b) selectively setting the pulse rate to the alternate pulse rate.

10. A method as claimed in claim 9, in which the step of determining an alternate pulse rate comprises the steps of:

    (a) evaluating a power spectrum corresponding to the data to determine which of a plurality of peaks in the power spectrum corresponds to the fundamental frequency;

    (b) generating an alternate pulse rate corresponding to the fundamental frequency; and

    (c) calculating a confidence level for the alternate pulse rate.

11. A method as claimed in claim 10, which includes the step of generating an initial pulse rate, the initial pulse rate being set to the alternate pulse rate in the setting step.

12. A method as claimed in claim 9, in which the step of determining an alternate pulse rate comprises the steps of:

    (a) identifying a sequence of waveform characteristics indicative of a waveform period;

    (b) averaging a number of successive waveform periods to determine an average waveform period; and

    (c) determining the alternate pulse rate from the average waveform period.

13. A method as claimed in claim 11 or claim 12, in which the step of determining an alternate pulse rate occurs periodically, the setting step occurring only where at least one condition is met.

14. A method as claimed in claim 13, in which the at least one condition is selected from the group comprising:

    (a) a confidence level corresponding to the filtered pulse rate is less than a first value;

    (b) the confidence level corresponding to the alternate pulse rate is greater than or equal to the first value;

    (c) a signal-to-noise ratio corresponding to the alternate pulse rate is greater than or equal to a second value;

    (d) the alternate pulse rate corresponds to a local maxima on an autocorrelation curve corresponding to the data; and

    (e) the alternate pulse rate is within a third value of a previously determined alternate pulse rate, the previously determined alternate pulse rate having been determined within a first interval.

15. A method as claimed in claim 14, in which conditions (a) to (e) are met before the pulse rate is set to the alternate pulse rate.

16. Apparatus for measuring a pulse rate of a patient using data corresponding to at least one wavelength of electromagnetic energy transmitted through tissue of the patient, comprising:

(a) a comb filter for isolating signal energy in the data corresponding to a fundamental frequency of the pulse rate and related higher frequency components thereof;
(b) means for determining a particular frequency which optimizes energy at an output of the comb filter; and
(c) means for generating a filtered pulse rate corresponding to the particular frequency.

**Patentansprüche**

1. Verfahren zum Messen einer Pulsgeschwindigkeit eines Patienten unter Verwendung von Daten, die mindestens einer Wellenlänge elektromagnetischer Energie entsprechen, welche durch Gewebe des Patienten übertragen wird, wobei das Verfahren die Schritte umfaßt:

a) Definieren eines Kammfilters, um Signalenergie in den Daten zu isolieren, welche einer fundamentalen Frequenz der Pulsgeschwindigkeit und damit in Beziehung stehenden höheren Frequenzkomponenten derselben entspricht;

b) Bestimmen einer speziellen Frequenz, welche die. Energie am Ausgang des Kammfilters optimiert; und

c) Erzeugen einer gefilterten Pulsgeschwindigkeit, die der speziellen Frequenz entspricht.

2. Verfahren nach Anspruch 1, in dem der Bestimmungsschritt die Energie am Ausgang des Kammfilters minimiert.

3. Verfahren nach Anspruch 1, in dem der Bestimmungsschritt die Energie am Ausgang des Kammfilters maximiert.

4. Verfahren nach Anspruch 1, in dem der Bestimmungsschritt umfaßt:

a) Berechnen eines quadrierten Rauschens der Daten;

b) Berechnen einer zweiten Ableitung des quadrierten Rauschens bezüglich der fundamentalen Frequenz;

c) Durchführen einer Newton-Raphson-Suche, um die spezielle Frequenz zu bestimmen.

5. Verfahren nach Anspruch 1, welches die Schritte einschließt:

a) Vergleichen von ausgewählten Proben der Pulsgeschwindigkeit mit einer erwarteten Meßcharakteristik;

b) Zuordnen eines variablen Gewichts zu jeder ausgewählten Probe auf der Basis des Vergleichsschritt, wodurch eine Mehrzahl von verschieden gewichteten Proben der Pulsgeschwindigkeit erzeugt wird;

c) Mitteln einer ersten Zahl der gewichteten Proben, um eine gefilterte Probe der Pulsgeschwindigkeit zu erhalten; und

d) Erzeugen einer gefilterten Pulsgeschwindigkeit aus einer Mehrzahl von gefilterten Proben.

6. Verfahren nach Anspruch 5, in dem die erste Zahl abhängig von den variablen Gewichten variiert, welche in dem Zuordnungsschritt zugeordnet werden.

7. Verfahren nach Anspruch 1, in dem das Verfahren vor dem Definierungsschritt weiter die Schritte umfaßt:

a) Verwenden des Logarithmus eines Signals, das für die mindestens eine Wellenlänge elektromagnetischer Energie repräsentativ ist, wodurch ein erstes Signal erzeugt wird;

b) Bandpass-Filtern des ersten Signals, wodurch ein zweites Signal erzeugt wird;

c) Normieren des zweiten Signals, wodurch ein drittes Signal erzeugt wird; und

d) Verwenden der Ableitung des dritten Signal, wodurch die Daten erzeugt werden.

8. Verfahren nach Anspruch 1, in dem ein Vertrauensniveau mit der Pulsgeschwindigkeit verbunden ist, wobei das Vertrauensniveau auf mindestens einem Qualitätsmaß beruht, das ausgewählt ist aus der Gruppe, welche die Pulssignalform, das Signal-Rausch-Verhältnis und die Korrelation zwischen mindestens zwei Wellenlängen elektromagnetischer Energie umfaßt.

9. Verfahren nach Anspruch 1, welches die Schritte einschließt:

a) Bestimmen einer alternativen Pulsgeschwindigkeit unter Verwendung eines alternativen Geschwindigkeits-Auffindungsmittels; und

b) Selektives Einstellen der Pulsgeschwindigkeit auf die alternative Pulsgeschwindigkeit.

10. Verfahren nach Anspruch 9, in dem der Schritt der Bestimmung einer alternativen Pulsgeschwindigkeit die Schritte umfaßt:

a) Bewerten eines Leistungsspektrums, das den Daten entspricht, um zu bestimmen, welcher einer Mehrzahl von Peaks in dem Leistungsspektrum der fundamentalen Frequenz entspricht;

b) Erzeugen einer alternativen Pulsgeschwindigkeit, die der fundamentalen Frequenz entspricht; und

c) Berechnen eines Vertrauensniveaus für die alternative Pulsgeschwindigkeit.

11. Verfahren nach Anspruch 10, welches den Schritt der Erzeugung einer anfänglichen Pulsgeschwindigkeit einschließt, wobei die anfängliche Pulsgeschwindigkeit auf die alternative Pulsgeschwindigkeit in dem Einstellungsschritt eingestellt wird.

12. Verfahren nach Anspruch 9, in dem der Schritt der Bestimmung einer alternativen Pulsgeschwindigkeit die Schritte umfaßt:

a) Identifizieren einer Reihenfolge von Wellenformencharakteristika, die eine Wellenformperiode anzeigen;

b) Mitteln einer Zahl von aufeinanderfolgenden Wellenformperioden, um eine durchschnittliche Wellenformperiode zu bestimmen; und

c) Bestimmen der alternativen Pulsgeschwindigkeit aus der mittleren Wellenformperiode.

13. Verfahren nach Anspruch 11 oder Anspruch 12, in dem der Schritt der Bestimmung einer alternativen Pulsgeschwindigkeit periodisch stattfindet, wobei der Einstellungsschritt nur dann stattfindet, wenn mindestens eine Bedingung erfüllt ist.

14. Verfahren nach Anspruch 13, in dem die mindestens eine Bedingung aus der Gruppe ausgewählt ist, welche umfaßt:

a) ein Vertrauensniveau, das der ersten gefilterten Pulsgeschwindigkeit entspricht, ist geringer als ein erster Wert;

b) das Vertrauensniveau, das der alternativen Pulsgeschwindigkeit entspricht, ist gleich oder größer als der erste Wert;

c) ein Signal-Rausch-Verhältnis, das der alternativen Pulsgeschwindigkeit entspricht, ist gleich oder größer als ein zweiter Wert;

d) die alternative Pulsgeschwindigkeit entspricht einem lokalen Maximum einer Autokorrelationskurve, welche den Daten entspricht; und

e) die alternative Pulsgeschwindigkeit liegt innerhalb eines dritten Wertes einer zuvor bestimmten alternativen Pulsgeschwindigkeit, wobei die zuvor bestimmte alternative Pulsgeschwindigkeit innerhalb eines ersten Intervalls bestimmt worden ist.

15. Verfahren nach Anspruch 14, in dem die Bedingungen

(a) bis (e) erfüllt sind, bevor die Pulsgeschwindigkeit auf die alternative Pulsgeschwindigkeit eingestellt wird.

16. Vorrichtung zum Messen einer Pulsgeschwindigkeit eines Patienten unter Verwendung von Daten, die mindestens einer Wellenlänge elektromagnetischer Energie entsprechen, die durch Gewebe des Patienten übertragen wird, welche umfaßt:

a) ein Kammfilter zur Isolierung von Signalenergie in den Daten, welche einer fundamentalen Frequenz der Pulsgeschwindigkeit und damit in Beziehung stehenden höheren Frequenzkomponenten derselben entsprechen;

b) Mittel zur Bestimmung einer speziellen Frequenz, welche Energie am Ausgang des Kammfilters optimiert;

c) Mittel zum Erzeugen einer gefilterten Pulsgeschwindigkeit, die der speziellen Frequenz entspricht.


**Revendications**

1. Procédé pour mesurer le pouls d'un patient en utilisant des données correspondant à au moins une longueur d'onde d'une énergie électromagnétique transmise par le tissu du patient, le procédé comprenant les étapes consistant à:

(a) définir un filtre en peigne pour isoler une énergie de signal dans les données correspondant à une fréquence fondamentale du pouls et des composantes associées ayant des fréquences plus élevées de ce pouls;
(b) déterminer une fréquence particulière qui optimise l'énergie au niveau d'une sortie du filtre en peigne; et
(c) produire un pouls filtré correspondant à la fréquence particulière.

2. Procédé selon la revendication 1, dans lequel l'étape de détermination rend minimale l'énergie à la sortie du filtre en peigne.

3. Procédé selon la revendication 1, dans lequel l'étape de détermination rend maximale l'énergie à la sortie du filtre en peigne.

4. Procédé selon la revendication 1, dans lequel l'étape de détermination comprend:

(a) le calcul d'un bruit élevé au carré pour les données;
(b) le calcul d'une dérivée seconde du bruit élevé au carré en rapport avec la fréquence fondamentale; et
(c) l'exécution d'une recherche de Newton-Raphson pour déterminer la fréquence particulière.

5. Procédé selon la revendication 1, qui inclut les étapes consistant à:

(a) comparer des échantillons sélectionnés du pouls à une caractéristique de mesure attendue;
(b) affecter un poids variable à chaque échantillon sélectionné sur la base de l'étape de comparaison, en produisant ainsi une pluralité d'échantillons pondérés différemment du pouls;
(c) former la moyenne d'un premier nombre des échantillons pondérés pour l'obtention d'un échantillon filtré du pouls; et
(d) produire un pouls filtré à partir d'une pluralité d'échantillons filtrés.

**6.** Procédé selon la revendication 5, selon lequel le premier nombre varie en réponse à des poids variables affectés lors de l'étape d'affectation.

**7.** Procédé selon la revendication 1, dans lequel, avant l'étape de définition, le procédé comprend en outre les étapes consistant à:

(a) prendre le logarithme d'un signal représentatif de la au moins une longueur d'onde d'énergie électromagnétique, en produisant ainsi un premier signal;
(b) réaliser un filtrage passe-bande du premier signal, en produisant ainsi un second signal;
(c) normaliser le second signal, en produisant ainsi un troisième signal; et
(d) prendre la dérivée du troisième signal, en produisant ainsi les données.

**8.** Procédé selon la revendication 1, selon lequel il est prévu un niveau de confiance associé au pouls, le niveau de confiance étant basé sur au moins une métrique de qualité sélectionnée dans le groupe comprenant la forme du signal de pouls, le rapport signal/bruit et la corrélation entre au moins deux longueurs d'onde d'énergie électromagnétique.

**9.** Procédé selon la revendication 1, qui inclut les étapes consistant à:

(a) déterminer un autre pouls en utilisant un autre dispositif de détermination du pouls; et
(b) régler sélectivement le pouls sur l'autre pouls.

**10.** Procédé selon la revendication 9, selon lequel l'étape de détermination d'un autre pouls comprend les étapes consistant à:

(a) évaluer un spectre de puissance correspondant aux données pour déterminer lequel d'une pluralité de pics dans le spectre de puissance correspond à la fréquence fondamentale;
(b) produire un autre pouls correspondant à la fréquence fondamentale; et
(c) calculer un niveau de confiance pour l'autre pouls.

**11.** Procédé selon la revendication 10, qui inclut l'étape consistant à produire un pouls initial, le pouls initial étant réglé sur l'autre pouls lors de l'étape de réglage.

**12.** Procédé selon la revendication 9, selon lequel l'étape de détermination d'un autre pouls comprend les étapes consistant à:

(a) identifier une séquence de caractéristiques de formes d'ondes indicatives d'une période de forme d'onde;
(b) former la moyenne d'un certain nombre de périodes successives de formes d'onde pour déterminer une période de forme d'onde moyenne; et
(c) déterminer l'autre pouls à partir de la période de forme d'onde moyenne.

**13.** Procédé selon la revendication 11 ou la revendication 12, dans lequel l'étape de détermination d'un autre pouls apparaît périodiquement, l'étape de réglage apparaissant uniquement là où au moins une condition est satisfaite.

**14.** Procédé selon la revendication 13, selon lequel la au moins une condition est sélectionnée dans le groupe comprenant:

(a) le niveau de confiance correspondant au pouls filtré est inférieur à une première valeur; à l'autre pouls.
(b) le niveau de confiance correspondant à l'autre pouls est supérieur ou égal à la première valeur;
(c) un rapport signal/bruit correspondant à l'autre pouls est supérieur ou égal à une seconde valeur;
(d) l'autre pouls correspond à un maximum local d'une courbe d'auto-corrélation, correspondant aux données; et
(e) l'autre pouls se situe en deçà d'une troisième valeur d'un autre pouls déterminé antérieurement, l'autre pouls déterminé antérieurement ayant été déterminé au cours d'un premier intervalle.

**15.** Procédé selon la revendication 14, selon lequel les conditions (a) à (e) sont satisfaites avant que le pouls soit réglé sur l'autre pouls.

**16.** Dispositif pour mesurer le pouls d'un patient en utilisant des données correspondant à au moins une longueur d'onde de l'énergie électromagnétique transmise à travers le tissu du patient, comprenant:

(a) un filtre en peigne pour isoler une énergie de signal dans les données correspondant à une fréquence fondamentale du pouls et des composantes associées ayant des fréquences plus élevées du pouls;
(b) des moyens pour déterminer une fréquence particulière, qui optimise l'énergie au niveau à la sortie du filtre en peigne; et
(c) des moyens pour produire un pouls filtré correspondant à la fréquence particulière.

FIG. 1

FIG. 2